# EUROPEAN PATENT APPLICATION

(11) **EP 3 854 364 A1**
(43) Date of publication of application: **28.07.2021**
(21) Application number: 19863027.9
(22) Date of filing: 18.09.2019
(51) Int. Cl.: A61F 13/515, A61F 13/475, A61F 13/511

(54) **ABSORBENT ARTICLE**

(30) Priority: 18.09.2018 JP 2018173481
(71) Applicant: DAIO PAPER CORPORATION, Shikokuchuo-shi Ehime 799-0492 (JP)
(72) Inventor: NAGASHIMA, Mariko, Sakura-shi, Tochigi 329-1411 (JP)
(74) Representative: Schön, Christoph
(86) International application number: PCT/JP2019/036464
(87) International publication number: WO 2020/059731

(57) **Abstract**

[Problem] To prevent separation and accompanying tearing of a front-surface sheet when non-heat-fusible fibers are used in the front-surface sheet.

[Solution] An absorber 4 is interposed between a front-surface sheet 3 and a back-surface sheet 2, and side sheets 7 are disposed on both side portions along the longitudinal direction. The front-surface sheet 3 contains non-heat-fusible fibers, and the side sheets 7 contain a heat-fusible resin. The front-surface sheet 3 is interposed between the laminated side sheets 7 on the outer side in the width direction than the absorber 4 whereby a side flap portion SF in which the side sheet 7, the front-surface sheet 3, the side sheet 7, and the back-surface sheet 2 are laminated in that order from the skin side is formed. A fusing portion 10 in which the sheets forming the side flap portion SF are integrally heat-fused is provided in a region of the side flap portion SF including at least the front and back ends of a sanitary napkin 1.

## Description

### [Technical Field]

The present invention relates to an absorbent article such as a sanitary napkin, a panty liner, or an incontinence pad, and more specifically, to an absorbent article which is provided with a front-surface sheet containing non-heat-fusible fibers such as cotton fibers and which prevents separation from front and back ends and accompanying tearing.

### [Background Art]

Conventionally, an absorbent article in which an absorber made of paper cotton such as crushed pulp is interposed between a liquid-impermeable back-surface sheet such as a polyethylene sheet or a polyethylene sheet-laminated nonwoven fabric and a liquid-permeable front sheet is known as the absorbent article.

The front-surface sheet forms a skin-contact surface, and thus is required to be flexible, obtain a dry feel even after absorption of an excreted liquid, and cause little irritation to a skin. Although a nonwoven fabric of synthetic fibers and a resin mesh sheet have been widely used in the field of absorbent articles, there is a problem that a front-surface sheet made of a synthetic fiber causes itching, rash, and the like. As a material solving this problem, a front-surface sheet made of cotton fiber (cotton) has been proposed.

An example of such an absorbent article in which cotton fiber is used for the front-surface sheet is disclosed in Patent Document 1 below. Patent Document 1 below discloses an absorbent article which includes a front-surface sheet containing non-heat-meltable fiber, a pair of side sheets disposed on a skin-facing surface of the front-surface sheet and spaced apart in a lateral direction, and a heat-meltable sheet disposed between the front-surface sheet and an absorber to overlap the side sheets with the front-surface sheet disposed therebetween, and in which the side sheets and the heat-meltable sheet are formed of synthetic fiber containing a thermoplastic resin, and a welding portion that joins the side sheets and the heat-meltable sheet together by thermal melting with the front-surface sheet disposed therebetween is formed between the side sheets and the heat-meltable sheet.

### [Citation List]

### [Patent Document]

Patent Document 1: Japanese Patent Application Publication No. 2013-66614

### [Summary of Invention]

### [Technical Problem]

However, in the absorbent article described in Patent Document 1, as shown in FIG. 11, since a welding portion 55 is formed such that a heat-meltable sheet 52 disposed between a front-surface sheet 50 and an absorber 51 is joined with side sheets 53 disposed on a skin-facing surface of the front-surface sheet 50 by thermal melting with the front-surface sheet 50 disposed therebetween, the front-surface sheet 50 is joined between the side sheets 53 and the heat-meltable sheet 52. However, since the welding portion 55 that joins these sheets does not reach a back-surface sheet 54, there is a problem that, when the side sheet 53 or the front-surface sheet 50 is pulled during wearing, the front-surface sheet 50 or the side sheet 53 is likely to float up together with the heat-meltable sheet 52 and be torn by being separated from the end. In particular, at the front and back ends of an absorbent article, since the end in which the side sheet, the front-surface sheet, and the heat-meltable sheet are laminated is exposed to the outer surface, separation from this portion or tearing is likely to occur. There is another problem that, when the absorbent article is curved in the front-back direction along the roundness in the front-back direction of the body, the difference in peripheral length between the inner periphery and the outer periphery due to the thickness of the thick absorber becomes large, and the laminated portion of the front-surface sheet and the back-surface sheet is likely to move and floating of the front-surface sheet is likely to occur.

Therefore, a main object of the present invention is to provide an absorbent article in which a non-heat-fusible fiber is used for a front-surface sheet, in which separation and accompanying tearing of the front-surface sheet are prevented.

### [Solution to Problem]

In order to solve the above-mentioned problems, the invention according to claim 1 provides an absorbent article in which an absorber is interposed between a front-surface sheet on a skin side and a back-surface sheet on a non-skin side, and side sheets are disposed on both side portions along a longitudinal direction, wherein the front-surface sheet contains non-heat-fusible fibers and the side sheets contain a heat-fusible resin, the front-surface sheet is interposed between a plurality of laminated side sheets on an outer side in a width direction than the absorber, whereby a side flap portion in which the side sheet, the front-surface sheet, the side sheet, and the back-surface sheet are laminated in that order from the skin side are laminated is formed, and a fusing portion in which the sheets forming the side flap portion are integrally heat-fused is provided in a region of the side flap portion including at least front and back ends of the absorbent article.

In the invention according to claim 1, a sheet containing non-heat-fusible fibers such as cotton fiber is used as the front-surface sheet, and a sheet containing a heat-fusible resin such as a synthetic fiber is used as the side sheet. The front-surface sheet is interposed between the plurality of laminated side sheets on the outer side in the width direction than the absorber whereby the side flap portion in which the side sheet, the front-surface sheet, the side sheet, and the back-surface sheet are laminated in that order from the skin side is formed. Further, the fusing portion in which the sheets forming the side flap portion are integrally heat-fused is provided in a region of the side flap portion including at least front and back ends of the absorbent article. In the fusing portion, the non-heat-fusible fibers contained in the front-surface sheet are not melted, but the side sheets containing the heat-fusible fibers disposed on the skin side and the non-skin side of the front-surface sheet, respectively, are melted by heating. The melted fibers are solidified by entering into the inter-fiber voids of the front-surface sheet whereby the side sheets, the front-surface sheet, and the back-surface sheet are integrally joined. As a result, it is possible to prevent the front-surface sheet from being separated from at least the front and back ends of the side flap portion of the absorbent article and prevent tearing of the sheets accompanied by the separation of the front-surface sheet.

The invention according to claim 2 provides the absorbent article according to claim 1, in which the fusing portion is provided in a front-side region and a back-side region of the absorbent article respectively.

In the invention according to claim 2, since the fusing portion is provided in the front-side region and the back-side region of the front-surface sheet of the absorbent article respectively where separation of the front-surface sheet is likely to occur, separation from the front and back ends of the absorbent article is prevented. Moreover, the fusing portion is not provided in the intermediate region in the front-back direction of the absorbent article between the front and back ends from the viewpoint of preventing the fusing portion from being cured to give discomfort to the wearer when wearing the absorbent article.

The invention according to claim 3 provides the absorbent article according to claim 1, in which the fusing portion is provided over an entire length in the longitudinal direction of the absorbent article connecting a front-side edge and a back-side edge of the absorbent article.

In the invention according to claim 3, in order to prevent separation of the front-surface sheet more reliably, the fusing portion is provided over the entire length in the longitudinal direction of the absorbent article connecting the front-side edge and the back-side edge of the absorbent article.

The invention according to claim 4 provides the absorbent article according to any one of claims 1 to 3, in which the fusing portion is continuously heat-fused or the fusing portion is intermittently heat-fused.

In the invention according to claim 4, the fusing portion is heat-fused continuously in order to enhance the joining strength of the fusing portion, or the fusing portion is intermittently heat-fused in a houndstooth pattern or a parallel linear pattern in order to reduce the deterioration of the wearability due to the curing of the fusing portion.

The invention according to claim 5 provides the absorbent article according to any one of claims 1 to 4, in which the side sheet disposed on the skin side of the front-surface sheet is composed of one layer or a plurality of layers, or an elastically stretchable member is disposed inside a laminated sheet laminated in a plurality of layers, and a three-dimensional gather that is erected to the skin side is formed in an intermediate portion in the longitudinal direction of the absorbent article.

In the invention according to claim 5, (1) an embodiment in which the side sheet is formed in one layer in order to simplify the structure, (2) an embodiment in which the side sheet is formed in a plurality of layers in order to enhance the effect of preventing leakage to the outer side in the width direction, and (3) an embodiment in which the three-dimensional gather is formed in order to further enhance the effect of preventing leakage to the outer side in the width direction are exemplified as the side sheet disposed on the skin side of the front-surface sheet.

The invention according to claim 6 provides the absorbent article according to claim 5, in which when the three-dimensional gather is formed by the side sheets, the laminated sheet of the side sheets is folded toward an outer side at the front and back ends of the absorbent article, and the skin side of the fusing portion is covered with the folded laminated sheet.

In the invention according to claim 6, when the three-dimensional gather is formed by the side sheets, the laminated sheet of the side sheets is folded toward an outer side at the front and back ends of the absorbent article, and the skin side of the fusing portion is covered with the folded laminated sheet. Therefore, it is possible to prevent the fusing portion which is cured by heat-fusing from making direct contact with the skin and prevent degradation of the wearing feeling.

The invention according to claim 7 provides the absorbent article according to any one of claims 1 to 6, in which a large number of openings penetrating through the skin side and the non-skin side are formed in the front-surface sheet, and the fusing portion is provided in a region overlapping the openings.

In the invention according to claim 7, when the front-surface sheet in which a large number of openings penetrating through the skin side and the non-skin side are formed is used, the fusing portion is provided in a region overlapping the openings. Therefore, since the side sheets laminated on the skin side and the non-skin side can be heat-fused through the openings, the joining strength can be enhanced further.

The invention according to claim 8 provides the absorbent article according to any one of claims 1 to 7, in which the side sheet disposed on the skin side of the front-surface sheet and the side sheet disposed on the non-skin side of the front-surface sheet are made of the same material.

In the invention according to claim 8, the side sheet disposed on the skin side of the front-surface sheet and the side sheet disposed on the non-skin side of the front-surface sheet are made of the same material. Therefore, the effect of thermal fusion can be further enhanced in such a way that both sheets can be melted at a constant temperature at the time of thermal fusion.

The invention according to claim 9 provides the absorbent article according to any one of claims 1 to 8, in which the side sheet disposed on the skin side of the front-surface sheet and the side sheet disposed on the non-skin side of the front-surface sheet are formed of one sheet that is folded so that side edges of the front-surface sheet are rolled up.

In the invention according to claim 9, the side sheets disposed on the skin side and the non-skin side of the front-surface sheet are formed of one sheet that is folded so that side edges of the front-surface sheet are rolled up. Therefore, since the manufacturing can be simplified, and the side edges of the front-surface sheet that easily retains water are completely covered with the side sheet, the body fluid retained in the front-surface sheet can be prevented from exudating from the side edges.

### [Advantageous Effects of Invention]

As described in detail above, according to the present invention, in an absorbent article in which non-heat-fusible fibers are used for the front-surface sheet, separation and accompanying tearing of the front-surface sheet can be prevented.

### [Brief Description of Drawings]

[FIG. 1] FIG. 1 is a partially cutaway development view of a sanitary napkin 1 according to the present invention.
[FIG. 2] FIG. 2 is a view taken along line II-II of FIG. 1.
[FIG. 3] FIG. 3 is a view taken along line III-III of FIG. 1.
[FIG. 4] FIG. 4 is an enlarged cross-sectional view of a fusing portion 10 (taken along line IV-IV in FIG. 1).
[FIG. 5] FIG. 5 is a plan view of a sanitary napkin 1 according to a modified example.
[FIG. 6] FIG. 6 is an enlarged plan view of the upper right portion of the sanitary napkin 1 showing the plan pattern of the fusing portion 10.
[FIG. 7] FIG. 7 is a cross-sectional view of a sanitary napkin 1 according to a first modified example.
[FIG. 8] FIG. 8 is a cross-sectional view of a sanitary napkin 1 according to a second modified example.
[FIG. 9] FIG. 9 is a cross-sectional view of the sanitary napkin 1, showing a processing procedure for a three-dimensional gather BS.
[FIG. 10] FIG. 10 is a cross-sectional view of a sanitary napkin 1 according to a third modified example.
[FIG. 11] FIG. 11 is an enlarged cross-sectional view showing a conventional absorbent article.

### [Description of Embodiments]

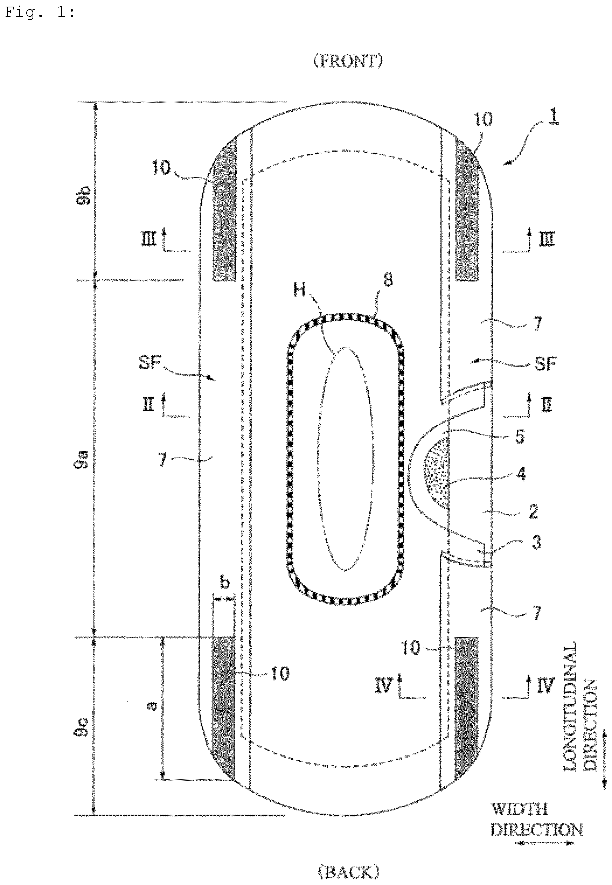

Hereinafter, embodiments of the present invention will be described in detail with reference to the drawings.
As shown in FIGS. 1 to 3, a sanitary napkin 1 according to the present invention is mainly composed of a liquid-impermeable back-surface sheet 2 made of a polyethylene sheet or the like, a front-surface sheet 3 that forms a skin-contact surface and allows rapid permeation of body fluid, an absorber 4 made of cotton-like pulp or synthetic pulp interposed between the two sheets 2 and 3, and side sheets 7 disposed on both side portions of the front-surface sheet 3 along a longitudinal direction, respectively. Around the absorber 4, the outer edge portions of the back-surface sheet 2 and the front-surface sheet 3 may be joined by an adhesive such as hot melt in at least a portion of the upper and lower edge portions thereof. Alternatively, the side sheets 7, the front-surface sheet 3 and the back-surface sheet 2 extending laterally further than the absorber 4 may be joined by an adhesive such as hot melt or a joining means such as heat seal at least a portion of both side edge portions thereof. However, in a section where a fusing portion 10 described later is provided, it is not necessary to perform joining using these joining means.

As shown in FIG. 1, the sanitary napkin 1 can be divided in the longitudinal direction such that a section corresponding to a body fluid discharge portion H of the wearer (that is, two sections when the sanitary napkin 1 is divided equally into four sections in the longitudinal direction) is a central region 9a, a front side thereof is a front-side region 9b, and a back side thereof is a back-side region 9c.

### <Back-surface sheet>

As the back-surface sheet 2, a sheet material having at least water-blocking properties such as polyethylene is used. However, in recent years, a sheet material having moisture permeability tends to be used from the viewpoint of preventing stuffiness. As the sheet material having the water-blocking properties and the moisture permeability, a microporous sheet obtained by melt-kneading inorganic filler in an olefin-based resin such as polyethylene or polypropylene to mold a sheet, and then stretching the sheet in a uniaxial direction or biaxial direction is suitably used.

The back-surface sheet 2 preferably contains heat-fusible fibers. As the heat-fusible fiber, arbitrary fiber which melts by heating and exhibits mutual adhesiveness can be used. This heat-fusible fiber may contain a single fiber or may be a composite fiber obtained by combining two or more types of synthetic resins. Specifically, it is possible to use polyolefin-based single fibers such as polyethylene, polypropylene and polyvinyl alcohol, a sheath-core type composite fiber or an eccentric sheath-core type composite fiber in which a sheath portion made of polyethylene terephthalate/polyethylene, polyethylene terephthalate/polypropylene, polypropylene/polyethylene, polyethylene terephthalate-ethylene/polypropylene copolymer, low melting point polyester-polyester, and the like is set to have a relatively low melting point, a split type composite fiber in which a part of each component made of polyethylene terephthalate/polypropylene, polyethylene terephthalate/nylon, and polypropylene/polyethylene is exposed to a surface, or a heat split type composite fiber split by heat shrinkage of one component made of a polyethylene terephthalate/ethylene-propylene copolymer.
In this case, the sheath-core type composite fiber is preferable in the case of placing importance on productivity and dimensional stability, and an eccentric type composite fiber is preferable when volume sense of the nonwoven fabric is emphasized. In addition, when flexibility is emphasized, and the split type composite fiber or the heat split type composite fiber is used, each component is easily divided into ultrafine fibers at the time of high pressure water flow treatment for entangling fibers.

### <Front-surface sheet>

The front-surface sheet 3 forms a skin-contact surface which is a portion covering the skin side of the absorber 4, and is formed of non-heat-fusible fibers. As the non-heat-fused fiber, arbitrary fiber that does not melt even when heated and does not exhibit adhesiveness between non-heat-fused fibers can be used. As the non-heat-fusible fiber, natural fibers such as cotton, pulp, silk, or lyocell, regenerated cellulose fibers such as rayon or cupra, or semi-synthetic fibers such as acetate may be used. Among these materials, natural fibers are preferably used because of their good touch. Among natural fibers, cotton fibers and lyocell fibers, which are particularly soft to the touch and have excellent water absorption and hygroscopicity, are preferably used. The front-surface sheet 3 may be formed of a spunlace nonwoven fabric containing 100 wt% of cotton fibers, or a laminated nonwoven fabric of a spunlace in which the skin side layer is made of 100 wt% of cotton fibers and heat-fusible fibers are disposed in the non-skin side layer. The spunlace nonwoven fabric has advantages that an adhesive is not used and the spunlace nonwoven fabric has flexibility.

As the cotton fiber, various cotton fibers such as raw cotton of a cotton plant, refined/bleached cotton fiber, cotton fiber dyed after being refined/bleached, refined/bleached absorbent cotton fiber, and recovered wool obtained by defibrating yarn or fabric can be used. Particularly, non-absorbent cotton slightly having water repellency even in a fiber state due to natural fat and oil of cotton wax attached to a surface of cotton fiber is preferably used.

It is preferable that a basis weight of the front-surface sheet 3 is set to 20 to 40 g/m², preferably 27 to 34 g/m², more preferably 29 to 32 g/m², and a thickness thereof is set to 0.25 to 0.50 mm, preferably 0.3 to 0.4 mm. The basis weight is calculated by measuring a weight of 5 cm × 4 cm × 10 sheets using an electronic balance and performing square meter conversion. Further, the thickness can be measured by a constant pressure thickness gauge digital type FFD-7 manufactured by Ozaki Seisakusho Co., Ltd.

In the front-surface sheet 3, a large number of openings penetrating the skin side and the non-skin side in the thickness direction may be provided to enhance liquid permeability. Specifically, the opening may be formed by supporting a fiber material on a mesh-like support in a hydroentanglement process at the time of manufacturing spunlace. In this case, by changing the condition of the mesh to be used, it is possible to adjust a size of each opening and an opening ratio. The openings may be formed by performing punching on the manufactured nonwoven fabric. The opening may be formed at least in the region where the fusing portion 10 described later is provided. However, it is preferable to form the opening in the body fluid discharge portion H and its vicinity in order to improve the water permeability in the body fluid discharge portion H, and it is particularly preferable to provide the opening in the entire front-surface sheet.

When a spunlace nonwoven fabric made of 100 wt% of cotton fibers coated with a water repellent agent and formed with a large number of openings penetrating through the skin side and the non-skin side is used as the front-surface sheet 3, it is preferable in that the soft touch is obtained, and skin trouble at the time of wearing such as itching or rash is rarely caused even after wearing for a long time. Moreover, it is also preferable in that the wearer rarely feels stuffiness before discharging the body fluid due to the hygroscopicity of the cotton fiber. At that time, the problematic surface liquid residue is sufficiently improved by applying a water repellent agent. Further, when a large number of openings are provided in the region of the front-surface sheet 3 including the body fluid discharge portion H, the body fluid quickly permeates the front-surface sheet 3 through the openings.

### <Absorbent 4>

The absorber 4 interposed between the back-surface sheet 2 and the front-surface sheet 3 is composed of, for example, cotton-like pulp and a water-absorbent polymer. The water-absorbent polymer is mixed in the pulp constituting the absorber, for example, as granular powder. Examples of the pulp include a cellulose fiber such as chemical pulp or dissolved pulp obtained from wood and an artificial cellulose fiber such as rayon or acetate. Soft wood pulp having a longer fiber length than that of hardwood pulp is suitably used in terms of function and price. The basis weight of the absorber 4 is preferably 290 to 1000 g/m², preferably 390 to 850 g/m², and more preferably 630 to 720 g/m².

In addition, the absorber 4 may be mixed with synthetic fibers. Examples of the synthetic fiber include a polyolefin-based fiber such as polyethylene or polypropylene, a polyester-based fiber such as polyethylene terephthalate or polybutylene terephthalate, a polyamide-based fiber such as nylon, copolymers thereof, and mixtures of two types. Further, composite fibers such as sheath-core type fibers in which fiber having a high melting point is used as a core and fiber having a low melting point is used as a sheath, side-by-side type fibers, and split type fibers can also be used. In the case of hydrophobic synthetic fibers, it is desirable that the synthetic fiber in which the surface is treated with a hydrophilic agent is used so as to have an affinity to body fluid.

Further, as the absorber 4, a polymer sheet in which a powder-granular superabsorbent polymer is supported between two upper and lower layers of hydrophilic sheets made of a nonwoven fabric, paper, or the like may be used. This polymer sheet has only a superabsorbent polymer supported between the two layers of sheets and does not have pulp fibers. If necessary, an intermediate sheet made of a hydrophilic nonwoven fabric or the like may be disposed between the pulp sheet and the front-surface sheet 3.

Further, as the absorber 4, a member in which an absorber composed of pulp fibers and a superabsorbent polymer is laminated on the skin side or the non-skin side of the polymer sheet may be used. The combination of the polymer sheet and the absorber composed of the pulp fiber and the superabsorbent polymer may be one layer each, or one or both of them may be a plurality of layers, and these layers may be laminated alternately.

Examples of the superabsorbent polymer include crosslinked polyacrylate, self-crosslinked polyacrylate, a saponified product of a crosslinked product of an acrylic acid ester-vinyl acetate copolymer, a crosslinked product of an isobutylene/maleic anhydride copolymer, a crosslinked product of polysulfonate, and a partially crosslinked water-swelling polymer such as polyethylene oxide or polyacrylamide. Among these examples, acrylic acid or acrylate salt-based one which is excellent in absorption amount and absorption rate is suitable. In the manufacturing process, an absorption ratio (absorption power) and an absorption rate of the superabsorbent polymer having absorbing performance mentioned above may be adjusted by adjusting crosslink density and crosslink density gradient.

When the absorber 4 contains the pulp fibers, the absorber 4 is preferably surrounded by a wrapping sheet 5 made of crepe paper, a nonwoven fabric, or the like for shape retention and polymer powder retention.

On the skin-side surface of the absorber 4, an embossed groove 8 which has an appropriate planar shape and is disposed so as to overlap at least the region corresponding to the body fluid discharge portion H of the wearer in the width direction and in which the front-surface sheet 3 and the absorber 4 are integrally recessed to the non-skin side is formed. By forming the embossed groove 8, the body fluid that has flowed into the embossed groove 8 can be reliably absorbed by the absorber 4. The planar shape of the embossed groove 8 may be, for example, a substantially elliptical shape in addition to a substantially oval shape as shown in FIG. 1. A known shape can be widely adopted as long as the embossed groove 8 is disposed so as to overlap at least the region corresponding to the body fluid discharge portion H of the wearer in the width direction.

### <Side sheet>

In the shown example, the front-surface sheet 3 is formed to be wider than the width of the absorber 4 by a predetermined width, and the side sheets 7 (a member different from the front-surface sheet 3) extending from both side portions of the front-surface sheet 3 are disposed on the outer side in the width direction of the front-surface sheet 3.

The side sheet 7 is formed of heat-fusible fibers similarly to the back-surface sheet 2. As the heat-fusible fiber, arbitrary fiber that melts by heating and exhibits mutual adhesiveness can be used. Similarly to the back-surface sheet 2, the heat-fusible fiber may be consisted of a single fiber or may be a composite fiber obtained by combining two or more types of synthetic resins. Specifically, it is possible to use polyolefin-based single fibers such as polyethylene, polypropylene and polyvinyl alcohol, a sheath-core type composite fiber or an eccentric sheath-core type composite fiber in which a sheath portion made of polyethylene terephthalate/polyethylene, polyethylene terephthalate/polypropylene, polypropylene/polyethylene, polyethylene terephthalate-ethylene/polypropylene copolymer, low melting point polyester-polyester, and the like is set to have a relatively low melting point, a split type composite fiber in which a part of each component made of polyethylene terephthalate/polypropylene, polyethylene terephthalate/nylon, and polypropylene/polyethylene is exposed to a surface, or a heat split type composite fiber split by heat shrinkage of one component made of a polyethylene terephthalate/ethylene-propylene copolymer.
In this case, the sheath-core type composite fiber is preferable in the case of placing importance on productivity and dimensional stability, and an eccentric type composite fiber is preferable when volume sense of the nonwoven fabric is emphasized. In addition, when flexibility is emphasized, and the split type composite fiber or the heat split type composite fiber is used, each component is easily divided into ultrafine fibers at the time of high pressure water flow treatment for entangling fibers.

As the side sheet 7, a nonwoven fabric material subjected to an appropriate water-repellent treatment or hydrophilic treatment depending on the purpose of preventing penetration of body fluid or the like or enhancing feeling of touch can be used.

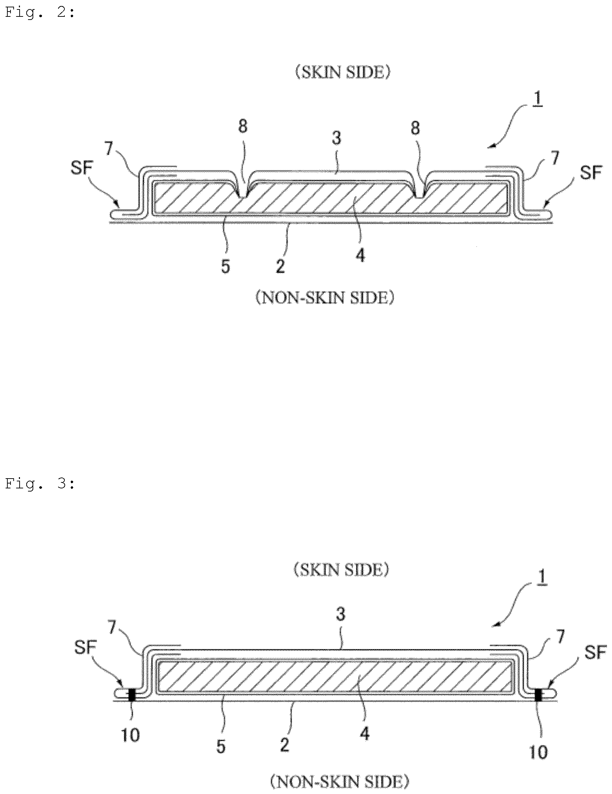

As shown in FIGS. 2 and 3, the side sheet 7 is disposed in a range from the inner position of the absorber 4 to the outer edge of the back-surface sheet 2 slightly beyond the side edge of the absorber in the intermediate portion in the width direction, and appropriate areas are attached by an adhesive such as hot melt. Adhesion of the side sheet 7 with an adhesive is preferably not performed at the portion where the fusing portion 10 described later is provided so as not to reduce the welding between the side sheets 7 due to thermal melting. Adhesion may be performed as long as it does not interfere with welding.

As shown in FIGS. 2 and 3, the side sheets 7 are laminated and disposed in a plurality of layers. In the shown example, the side sheets 7 are laminated in two layers by being folded at the end position in the width direction of the sanitary napkin 1. A side portion of the front-surface sheet 3 extending from the inside in the width direction is interposed between the side sheets 7 laminated in two layers. As a result, a side flap portion SF in which the side sheet 7, the front-surface sheet 3, the side sheet 7, and the back-surface sheet 2 are laminated in that order from the skin side is formed on the outer side in the width direction than the absorber 4. That is, in the side flap portion SF, the skin surface side and the non-skin surface side of the front-surface sheet 3 are covered with the side sheet 7 respectively.

Both the side sheet 7 disposed on the skin side of the front-surface sheet 3 and the side sheet 7 disposed on the non-skin side of the front-surface sheet 3 are separated at the center in the width direction of the sanitary napkin 1 and are disposed along the longitudinal direction on both side portions of the sanitary napkin 1. When the side sheet 7 disposed on the skin side of the front-surface sheet 3 is extended to the center of the sanitary napkin 1 in the width direction, the softness of the front-surface sheet 3 made of cotton fibers or the like is lost, which is not preferable. Further, when the side sheet 7 disposed on the non-skin side of the front-surface sheet 3 is extended to the center in the width direction of the sanitary napkin 1, the side sheet 7 is interposed between the front-surface sheet 3 and the absorber 4, and the transfer of body fluid from the front-surface sheet 3 to the absorber 4 may be reduced, which is not preferable.

In the shown example, one side sheet 7 is laminated in two layers by being folded at the end position in the width direction of the sanitary napkin 1 so that the side edges of the front-surface sheet 3 are rolled up. However, the side sheet 7 may be laminated in two layers by disposing separate side sheets 7 on the skin-side surface and the non-skin-side surface of the front-surface sheet 3. When separate side sheets 7 are arranged, it is desirable to use the same material. However, the side sheets 7 may be made of different materials in expectation of effects such as enhancing the joining strength between the front-surface sheet 3 on the skin side and the back-surface sheet 2 on the non-skin side by blending more fibers having a lower melting temperature in the side sheet 7 on the non-skin side than the side sheet 7 disposed on the skin side of the front-surface sheet 3.

The side sheet 7 disposed on the skin side of the front-surface sheet 3 and the side sheet 7 disposed on the non-skin side of the front-surface sheet 3 are preferably formed of the same material. By forming the side sheets 7 using the same material, the effect of thermal fusion can be further enhanced in such a way that both can be thermally melted at a constant temperature at the time of thermal fusion of the fusing portion 10 described later, and the thermal-melted side sheets 7 can easily permeate the front-surface sheet 3 and the sheets are easily joined.

As shown in FIGS. 1 to 4, the side sheet 7 disposed on the skin side of the front-surface sheet 3 and the side sheet 7 disposed on the non-skin side of the front-surface sheet 3 are preferably formed of a single sheet folded so that the side edges of the front-surface sheet 3 are rolled up. As a result, the manufacturing can be simplified, and the side edges of the front-surface sheet 3 made of cotton fiber or the like that easily retains water are completely covered with the side sheet 7. Therefore, the body fluid retained in the front-surface sheet 3 can be prevented from exudating from the side edges.

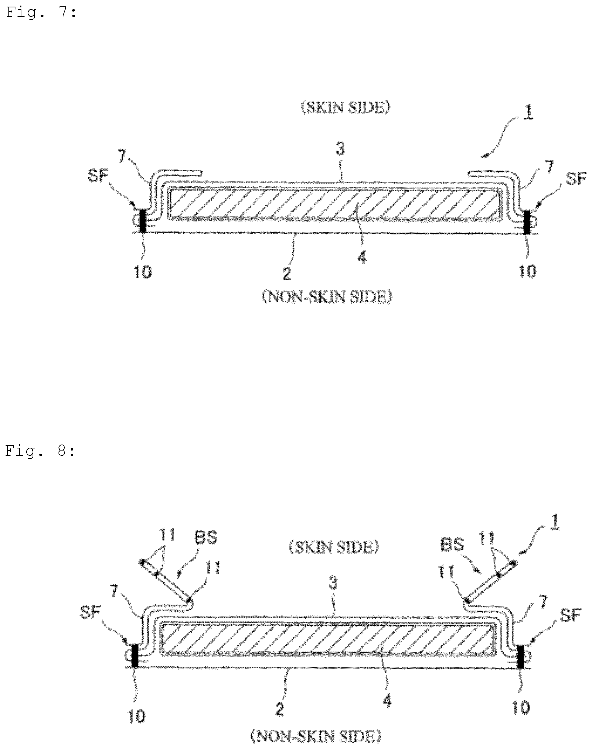

In the example shown in FIGS. 1 to 3, the side sheet 7 disposed on the skin side of the front-surface sheet 3 is composed of one layer, but may be a plurality of layers as described in detail later (see FIG. 7). By disposing elastically stretchable members 11 inside a double-folded double sheet, a three-dimensional gather BS that is erected on the skin side may be formed at the intermediate portion in the longitudinal direction of the sanitary napkin 1 (see FIG. 8).

### <Fusing portion>

In the sanitary napkin 1, as shown in FIGS. 1, 3 and 4, the fusing portion 10 in which the sheets (the side sheet 7, the front-surface sheet 3, the side sheet 7, and the back-surface sheet 2 in that order from the skin side) forming the side flap portion SF are integrally heat-fused is provided in a region of the side flap portion SF including at least the front and back ends of the sanitary napkin 1. In the fusing portion 10, the non-heat-fusible fibers of the front-surface sheet 3 are not melted, but the side sheets 7 containing the heat-fusible resins laminated on the skin side and the non-skin side of the front-surface sheet 3, respectively, are melted by heating. The melted fibers are solidified by entering into the inter-fiber voids of the front-surface sheet 3 and a large number of openings formed in the front-surface sheet 3. In this way, the side sheets 7 and the front-surface sheet 3 are integrally joined, and the side sheet 7 disposed on the non-skin side of the front-surface sheet 3 is joined with the back-surface sheet 2. As a result, all the sheets forming the side flap portion SF are integrally joined by the fusing portion 10. In particular, when a large number of openings are formed in the front-surface sheet 3, since the melted heat-fusible fibers of the molten side sheets 7 disposed on the skin side and the non-skin side of the front-surface sheet 3 can be joined through the openings, the sheets can be joined more firmly. Since the joining by the joining portion 10 is structural joining by melting and solidifying the heat-fusible fibers constituting the side sheets 7, the surfaces can be joined more firmly than the adhesion of two layers by application of an adhesive, and separation of the sheets can be prevented reliably.

The fusing portion 10 is formed in the side flap portion SF in which the side sheet 7, the front-surface sheet 3, the side sheet 7, and the back-surface sheet 2 in which the absorber 4 is not disposed are laminated in that order from the skin side in a region that does not overlap in the thickness direction of the absorber 4 on the outer side in the width direction than the absorber 4.

Further, the fusing portion 10 is formed in a region including at least the front and back ends of the sanitary napkin 1. The expression "including the front and back ends of the sanitary napkin 1" means that the fusing portion 10 is formed starting from the edges in the longitudinal direction (the front-back direction) of the sanitary napkin 1. As a result, the edges of the front-surface sheet 3 disposed on the front and back edges of the sanitary napkin 1 are firmly fixed to the side sheets 7, and separation from the edges can be reliably prevented.

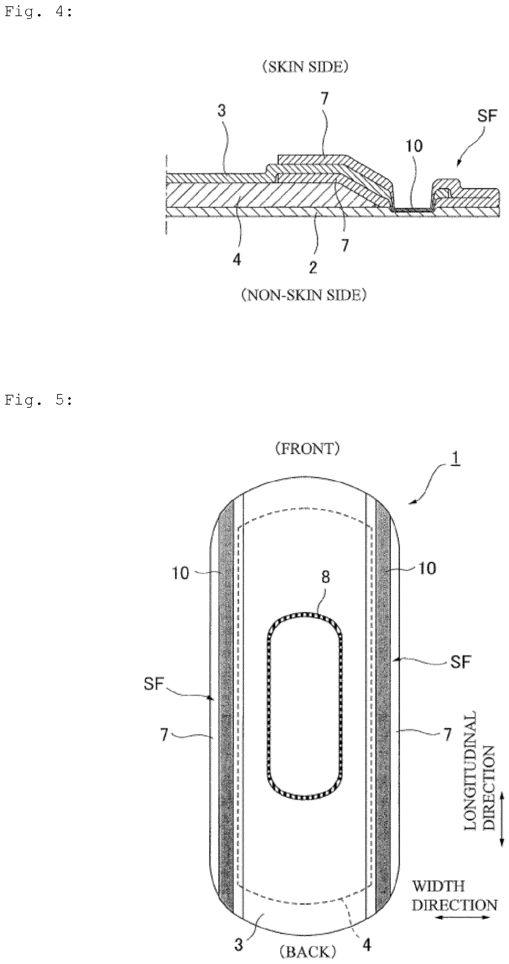

In order to form the fusing portion 10, as shown in FIG. 4, the side sheet 7, the front-surface sheet 3 and the back-surface sheet 2 are introduced between an embossing roll having embossed protrusions on the surface and an anvil roll having a flat surface and are pressed from the skin side of the side sheet 7, and are heated to a temperature equal to or higher than the melting point of the heat-fusible fibers constituting the side sheet 7 and the back-surface sheet 2 to melt the heat-fusible fibers. In the fusing portion 10, the side sheet 7, the front-surface sheet 3 and the back-surface sheet 2 are pressed to the non-skin side, and a recess recessed to the non-skin side is formed in the skin-side surface of the side sheet 7.

As one embodiment of the fusion portion 10, as shown in FIG. 1, the fusing portion 10 may be provided in the front-side region 9b and the back-side region 9c of the sanitary napkin 1, and may be not provided in the central region 9a. As a result, separation at the front and back ends of the front-surface sheet 3 of the sanitary napkin 1 where separation is likely to occur can be prevented, and the fusing portion 10 cured by thermal melting in the central region 9a can be prevented from coming into contact with the skin surface and giving discomfort and skin troubles when wearing the sanitary napkin 1.

Further, as another example of the fusing portion 10, as shown in FIG. 5, the fusing portion 10 may be provided in the entire length in the longitudinal direction of the sanitary napkin 1 connecting the front-side edge and the back-side edge of the sanitary napkin 1. As a result, the joining strength of the fusing portion 10 can be increased, and separation of the front-surface sheet 3 can be prevented over the entire length of the sanitary napkin 1. Further, since the heat-fused portion is formed on both side portions of the absorber 4 over the entire length of the sanitary napkin 1, leakage from the side edges of the sanitary napkin 1 can be reliably prevented.

The dimensions of the fusing portion 10 will be described with reference to FIG. 1. The length a of the sanitary napkin 1 in the longitudinal direction is preferably 8 mm or more from the front and back edges of the sanitary napkin 1, respectively. If the length is shorter than 8 mm, the joining strength of the fusing portion 10 is low, and separation is likely to occur. When the fusing portion 10 is provided in the front-side region 9b and the back-side region 9c respectively, the length should be set such that the inner end of the sanitary napkin 1 in the longitudinal direction does not cover the central region 9a. Further, as described above, the fusing portion 10 may be provided over the entire length of the sanitary napkin 1.

The width b of the fusing portion 10 is smaller than the width of the side flap portion SF, and specifically, is preferably 2 to 15 mm. If the width is smaller than 2 mm, the joining strength of the fusing portion 10 is low, and separation is likely to occur. If the width is larger than 15 mm, the fusing portion 10 becomes too hard and the wearing feeling deteriorates. The side sheet 7, the front-surface sheet 3, and the back-surface sheet 2 are extended by a predetermined length to the outer side in the width direction than the absorber 4 so that the width b of the fusing portion 10 can be secured. The fusing portion 10 is provided at an intermediate portion in the width direction of the side flap portion SF on the outer side in the width direction than the absorber 4, which does not reach the side edge of the sanitary napkin 1. That is, a flap portion in which at least two layers of the side sheet 7 and the back-surface sheet 2 are laminated and the fusing portion 10 is not formed is formed on the outer side in the width direction from the fusing portion 10. Therefore, when the sanitary napkin 1 is worn, the side edges of the sanitary napkin 1 become soft against the skin, and degradation of the wearing feeling is prevented.

As shown in FIG. 1, the fusing portion 10 can be formed by continuously pressurizing and heat-fusing a predetermined region over the entire surface. As a result, the joining strength of the fusing portion 10 is increased, and separation of the front-surface sheet 3 can be reliably prevented.

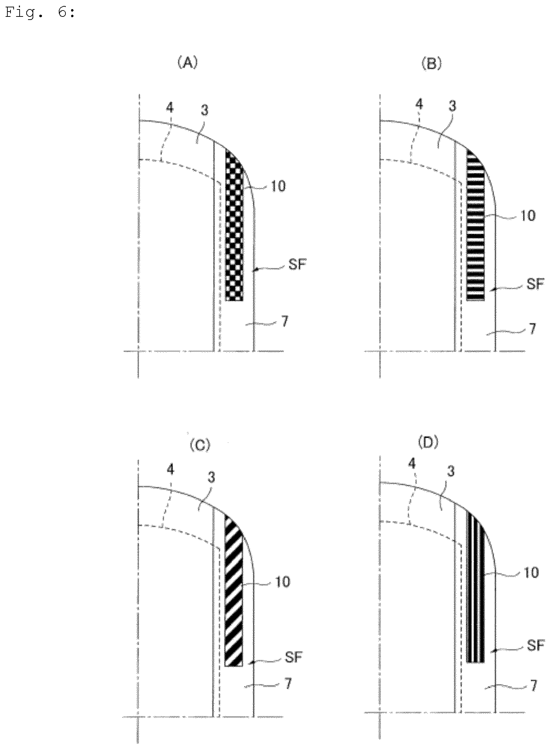

Further, as shown in FIG. 6, the fusing portion 10 may be formed by intermittently pressurizing and heat-fusing a predetermined region. In FIG. 6, the dark portion is a pressurized and heat-fused portion, and the other portion is an intermittent portion that has not been pressurized and heat-fused. Specifically, FIG. 6(A) is a houndstooth pattern, FIG. 6(B) is a striped pattern along the width direction, FIG. 6(C) is a striped pattern along the diagonal direction, and FIG. 6(D) is a striped pattern along the longitudinal direction. By intermittently forming the fusing portion 10, it is possible to reduce the deterioration of the wearability due to the curing of the fusing portion 10. When the fusing portion 10 is formed intermittently, the area of the pressurized and heat-fused portion with respect to the total area of the fusing portion 10 is 50% or more, preferably 50 to 70% in order to secure the joining strength.

As described above, the side sheet 7 disposed on the skin side of the front-surface sheet 3 may be composed of one layer as shown in FIG. 3. In this case, the structure can be simplified and the manufacturing cost can be reduced, but the effect of preventing lateral leakage is poor.

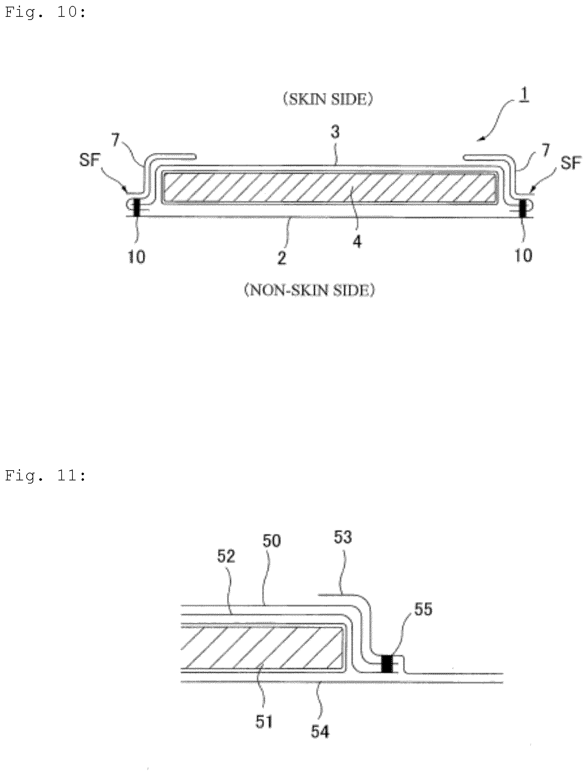

On the other hand, as shown in FIG. 7, the side sheets 7 disposed on the skin side of the front-surface sheet 3 may be laminated in a plurality of two or more layers. As a result, the side flap portion SF is formed high on the skin side, and the effect of preventing leakage to the outer side in the width direction can be enhanced. When a plurality of layers of side sheets 7 is laminated on the skin side of the front-surface sheet 3 in this way, the fusing portion 10 may include at least the side sheet 7 on the lowest layer among the side sheets 7 disposed on the skin side of the front-surface sheet 3, and the side sheets 7 of the upper layers may be attached by a hot melt adhesive or the like so as to cover the skin side of the fusing portion 10 as shown in FIG. 10. As a result, since the skin side of the fusing portion 10 is covered with the side sheet 7, the fusing portion 10 does not come into direct contact with the skin, and the skin contact is improved.

Further, as shown in FIG. 8, one or a plurality of (three in the shown example) threadlike elastically stretchable members 11 of which both end positions or appropriate positions in the longitudinal direction are fixed to an intermediate portion in the height direction may be disposed inside the double sheets in which the side sheet 7 is double-folded, and may be attached to the back-surface sheet 2 side (the skin-side surface of the side sheet 7) in a state where the double sheet portion is laminated by being folded once toward the outer side at the front and back ends. In this way, linear three-dimensional gathers BS that are erected to the skin side while being inclined to the outer side at the intermediate portion in the longitudinal direction of the sanitary napkin 1 may be formed in pair of left and right. As a result, it is possible to further enhance the effect of preventing leakage to the outer side in the width direction.

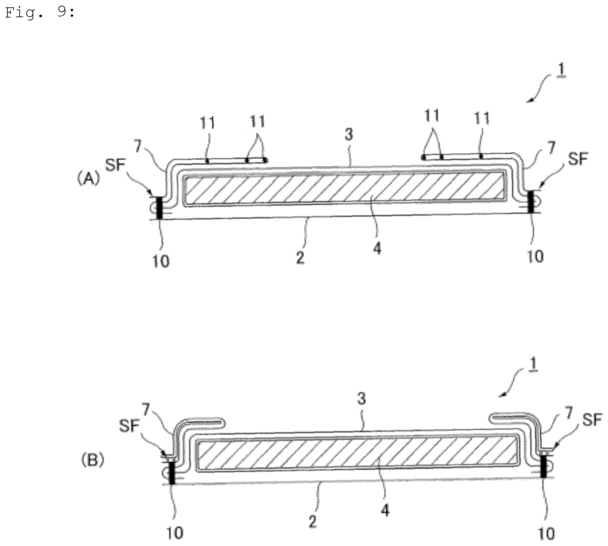

In order to form the three-dimensional gather BS shown in FIG. 8, first, as shown in FIG. 9(A), the fusing portion 10 is formed at a predetermined position in a state where the double sheet is extended to the inner side in the width direction. After that, as shown in FIG. 9(B), the double sheet portion is folded once toward the outer side and is attached to the back-surface sheet 2 side (the skin-side surface of the side sheet 7) at the front and back ends of the sanitary napkin 1 by a hot melt adhesive or the like. As a result, the intermediate portion in the longitudinal direction becomes the three-dimensional gather BS that is erected to the skin side as shown in FIG. 8.

At this time, as shown in FIG. 9(B), the double sheet portion that has been folded once toward the outer side preferably covers the skin side of the fusing portion 10 at the front and back ends of the sanitary napkin 1. As a result, it is possible to prevent the fusing portion 10 cured by thermal melting from coming into direct contact with the skin, and it is possible to prevent a decrease in wearing feeling.

### [Reference Signs List]

- 1:: Sanitary napkin
- 2:: Back-surface sheet
- 3:: Front-surface sheet
- 4:: Absorber
- 5:: Wrapping sheet
- 7:: Side sheet
- 8:: Embossed groove
- 9a:: Central region
- 9b:: Front-side region
- 9c:: Back-side region
- 10:: Fusing portion
- 11:: Threadlike elastically stretchable member

## Claims

1. An absorbent article in which an absorber is interposed between a front-surface sheet on a skin side and a back-surface sheet on a non-skin side, and side sheets are disposed on both side portions along a longitudinal direction, wherein
the front-surface sheet contains non-heat-fusible fibers and the side sheets contain a heat-fusible resin,
the front-surface sheet is interposed between a plurality of laminated side sheets on an outer side in a width direction than the absorber, whereby a side flap portion in which the side sheet, the front-surface sheet, the side sheet, and the back-surface sheet are laminated in that order from the skin side are laminated is formed, and
a fusing portion in which the sheets forming the side flap portion are integrally heat-fused is provided in a region of the side flap portion including at least front and back ends of the absorbent article.

2. The absorbent article according to claim 1, wherein
the fusing portion is provided in a front-side region and a back-side region of the absorbent article respectively.

3. The absorbent article according to claim 1, wherein
the fusing portion is provided over an entire length in the longitudinal direction of the absorbent article connecting a front-side edge and a back-side edge of the absorbent article.

4. The absorbent article according to any one of claims 1 to 3,
wherein
the fusing portion is continuously heat-fused or the fusing portion is intermittently heat-fused.

5. The absorbent article according to any one of claims 1 to 4, wherein
the side sheet disposed on the skin side of the front-surface sheet is composed of one layer or a plurality of layers, or an elastically stretchable member is disposed inside a laminated sheet laminated in a plurality of layers, and a three-dimensional gather that is erected to the skin side is formed in an intermediate portion in the longitudinal direction of the absorbent article.

6. The absorbent article according to claim 5, wherein
when the three-dimensional gather is formed by the side sheets, the laminated sheet of the side sheets are folded toward an outer side at the front and back ends of the absorbent article, and the skin side of the fusing portion is covered with the folded laminated sheet.

7. The absorbent article according to any one of claims 1 to 6, wherein
a large number of openings penetrating through the skin side and the non-skin side are formed in the front-surface sheet, and the fusing portion is provided in a region overlapping the openings.

8. The absorbent article according to according to any one of claims 1 to 7, wherein
the side sheet disposed on the skin side of the front-surface sheet and the side sheet disposed on the non-skin side of the front-surface sheet are made of the same material.

9. The absorbent article according to any one of claims 1 to 8, wherein
the side sheet disposed on the skin side of the front-surface sheet and the side sheet disposed on the non-skin side of the front-surface sheet are formed of one sheet that is folded so that side edges of the front-surface sheet are rolled up.
